# EUROPEAN PATENT SPECIFICATION

(11) **EP 3 183 570 B1**
(45) Date of publication and mention of the grant of the patent: **25.07.2018**
(21) Application number: 15750747.6
(22) Date of filing: 17.08.2015
(51) Int. Cl.: G01N 33/00, G01N 33/28, G01N 21/77

(54) **HYDROGEN SENSOR HAVING A PROTECTION LAYER**
WASSERSTOFFSENSOR MIT SCHUTZSCHICHT
CAPTEUR D'HYDROGÈNE DOTÉ D'UNE COUCHE DE PROTECTION

(30) Priority: 19.08.2014 EP 14181426
(43) Date of publication of application: 28.06.2017
(73) Proprietor: ABB Schweiz AG, 5400 Baden (CH)
(72) Inventor: PANELLA, Barbara, d-69469 Weinheim (DE); VAN MECHELEN, Jacobus Lodevicus Martinus, CH-8104 Regensdorf (CH); WESTERWAAL, Ruud Johannes, NL-1768 BX Barsingerhorn (NL); DAM, Bernhard, NL-2595 GE Den Haag (NL)
(74) Representative: ABB Patent Attorneys
(86) International application number: PCT/EP2015/068826
(87) International publication number: WO 2016/026803

(56) References cited:
- EP-A1- 2 105 734
- EP-A1- 2 559 778
- WO-A1-2007/126313
- WO-A1-2009/126568
- US-A- 5 606 170
- US-A- 5 640 470
- US-A- 5 783 152
- US-A- 6 041 643
- US-A1- 2012 085 145

## Description

Aspects of the present disclosure relate to a sensor for gas, which has a protective layer to shield the sensor from unwanted components in a fluid surrounding the sensor, in particular to a gas sensor having a protective coating, more particularly to a solid state metal-based hydrogen sensor having such a coating.

### Technical background

Solid state hydrogen sensors possess typically a catalytic layer which has the function to dissociate the hydrogen molecules into hydrogen atoms. The hydrogen atoms diffuse into the sensing element upon which it changes its physical properties, which can be correlated to the hydrogen concentration of the probed medium. This change can be for example a variation of the resistivity or optical properties of the sensing element and is recorded with an electrical or optical measurement, respectively.

Some gases, for example H₂S, may cause poisoning of this catalytic layer which is reflected in the degradation of the sensing properties. This can lead to a reduction in the lifetime of the sensor and/or to a degradation of measurement accuracy, in particular to wrong detector readings due to a permanent or reversible poisoning of the sensor with such gases. It is therefore important to protect such sensors from contact with poisoning gases. This can be achieved with a protective coating on top of the catalytic layer which allows diffusion of hydrogen but blocks poisoning gases.

A possible application of interest where a protective coating is needed is the detection of hydrogen dissolved in transformer oil. The hydrogen can be taken as an early indicator of transformer faults. It is clear that while an application for measuring dissolved hydrogen in a liquid, particularly in oil is mentioned, the protective layer is also suitable for the use of a sensor for detection in a gas phase.

WO2009/126568 refers to a protective coating for a solid state sensor having a catalytic layer, as e.g. consisting of Pd alloys. The protective layer contains silicon dioxide to protect from contaminating gases, in particular oxygen, which may lead to an oxidation of parts of the sensor.

US2010/0014151 A1 refers to a hydrogen sensor based on a switchable mirror device having a protective coating which is water impermeable. As a protection coating, PTFE is mentioned. The coating has the function to block the transport of liquids, such as water, to the sensor.

EP 2 105 734 A1 discloses a sensor having a gas-sensitive layer with a surface area and an electrical potential sensor capacitively coupled with the area via an air gap, the area being covered by an electric insulating coating that is permeable for hydrogen and oxygen gases. The coating contains polymethylmethacrylate.

WO 2009/126568 A1 discloses a protective coating for sustaining long term performance of a solid-state sensor of a gaseous constituent in a fluid stream. The sensor comprises a catalyst layer for promoting electrochemical dissociation of the gaseous constituent, the coating comprises at least one layer of silicon dioxide.

US 5783152 A discloses a sensor probe device for monitoring of hydrogen gas concentrations and temperatures by the same sensor probe. The sensor probe is constructed using thin-film deposition methods for the placement of a multitude of layers of materials sensitive to hydrogen concentrations and temperature on the end of a light transparent lens located within the sensor probe.

In view of the above and other factors, there is a need for the present invention.

### Summary of the invention

In view of the above, a hydrogen sensor according to claim 1, a detection system according to claim 11, a device according to claim 12, and a method according to claim 13 are provided. Further advantages, features, aspects and details that can be combined with embodiments described herein are evident from the dependent claims, the description and the drawings.

According to a first aspect a hydrogen sensor for detecting hydrogen in a fluid of oil in physical contact with the sensor is provided. The sensor comprises a sensing element, and a first protection layer, provided to prevent contact of the sensing element with a sensor poisoning gas in the fluid, wherein the first protection layer comprises PMMA (polymethyl methacrylate).

According to a further aspect, a detection system for hydrogen in fluids of oil is provided. The detection system comprises a hydrogen sensor for detecting hydrogen in a fluid in physical contact with the sensor, wherein the sensor comprises a sensing element, and a first protection layer, provided to prevent contact of the sensing element with a sensor poisoning gas, wherein the first protection layer comprises PMMA, a temperature sensor, a light source, a light detection device, and a control unit, wherein light from the light source is coupled into the sensor, light reflected by the sensing element of the sensor is detected by the light detection device, and wherein the control unit processes an output signal of the light detection device, determines a hydrogen concentration, and delivers a respective signal.

According to a yet further aspect, a device for electric power generation, transmission, or distribution is provided, comprising an oil volume, and a detection system for hydrogen in the oil, wherein the detection system comprises a hydrogen sensor for detecting hydrogen in a fluid in physical contact with the sensor, wherein the sensor comprises a sensing element, and a first protection layer, provided to prevent contact of the sensing element with a sensor poisoning gas, wherein the first protection layer comprises PMMA, a temperature sensor, a light source, a light detection device, and a control unit, wherein light from the light source is coupled into the sensor, light reflected by the sensing element of the sensor is detected by the light detection device, and wherein the control unit processes an output signal of the light detection device, determines a hydrogen concentration, and delivers a respective signal.

According to a further aspect, a method for producing a hydrogen sensor for detecting hydrogen in a fluid of oil in physical contact with the hydrogen sensor is provided. The method comprises providing a sensing element, and providing a first protection layer comprising PMMA on the sensing element.

It was found that the typical hydrogen concentrations in transformer oil can be more than 10 times smaller than the concentration of carbon monoxide (CO). As CO is a gas which is poisonous for sensors with a Pd-based catalyst, the protection of the hydrogen sensor from CO in a fluid to be probed is of high importance for its technical application.

The advantages of sensors with a protection coating according to embodiments are for example, that the sensor is protected from poisoning induced by carbon monoxide in an effective manner. Further, the sensor is protected from liquids, such as oil, and can hence also be used directly for liquid-dissolved hydrogen detection in applications such as power transformer diagnostics. Also, protection layers comprising PMMA can be combined in multilayer approaches with other coatings such as PTFE (polytetrafluoroethylene), SiO₂ (silicon dioxide), or Aluminium Oxide.

### Brief description of the Figures

More details will be described in the following with reference to the figures, wherein
- Fig. 1: is a schematic view of an examplary hydrogen sensor useful for understanding the invention;
- Fig. 2: is a schematic view of a hydrogen sensor according to embodiments;
- Fig. 3: is a schematic view of a yet further hydrogen sensor according to embodiments;
- Fig. 4: is a diagram of a test with two conventional sensors;
- Fig. 5: is a diagram of a test with a sensor according to embodiments;
- Fig. 6: is a schematic view of a hydrogen sensor according to embodiments;
- Fig. 7: is a schematic view of a hydrogen detection system according to embodiments;
- Fig. 8: is a schematic view of an electrical device according to embodiments.

### Detailed Description of Aspects of the Invention

As used herein, metal alloys defined by a formula with percent values typically adding to 100 percent, such as, for example, Mg₅₂Ni₂₀Zr₂₈, are meant to include also substances with a composition deviating from that with the exact numbers provided. Typically, alloys having a composition wherein each number, independently from one another, has a tolerance of +/-15 percent, are still regarded to fall under the metal alloy provided by provision of the exact formula, such as the example above, also if the single numbers do not add up to 100 in total. Also, as used herein, such alloys may comprise further, non-named substances such as chemical elements of smaller amounts, such as up to about 2 percent each, but not more than about 10 percent in total.

As used herein, the term "fluid" is intended to be representative for liquids. It is, however, specifically used to be representative of an insulation liquid, an oil, which is part of the insulation and/or cooling system of an electrical device, more particularly of a power transformer.

In the following, the present invention described in detail. First, some general possible aspects relating to the sensor assembly are described. The sensor assembly is adapted for sensing a status condition of an insulation-liquid-filled electrical equipment. Herein, electrical equipment refers to any equipment such as shunt reactors, bushings and transformers. The invention is particularly suited for the insulation liquid being insulation oil, be it on a mineral basis or from organic sources, such as palm oil. The invention is further particularly suited for the electrical equipment being a transformer, such as a power or distribution transformer, and more particularly for an oil-filled transformer.

According to the present invention, the protective coating of PMMA is applied to a fiber-optic hydrogen sensor. Therein, a Pd alloy can serve both as a catalytic layer and a sensing layer. The PMMA coating is deposited on top of the Pd alloy layer, which is deposited at the end of an optical fiber. The sensor may have e.g. a Ti adhesion layer between the optical fiber and the sensing layer. Other possible fiber optic sensor configurations are related to a multilayer system where the catalytic layer, typically a palladium alloy, is deposited onto a different sensing layer (e.g. MgTi), and the PMMA covers the catalytic layer or both. The optimal thickness of the protective layer can be chosen in a way for having a fast diffusion time of hydrogen through the coating and maximum blocking of CO. Typical coating thicknesses can be e.g. from about 100 nm to about 3.000, more typically from about 150 nm to about 2.000 nm, for a high diffusion rate of hydrogen, but larger thicknesses can be selected if a fast response time for hydrogen detection is not required, and if, for example, a protection is required against significantly higher CO concentrations than are described herein. The improved protection is achieved due to the combination of PMMA with other coating materials. For instance, state of the art shows that PTFE (polytetrafluoroethylene) coatings show good properties for the protection on solid state hydrogen sensors towards water. This coating, however, does not protect the sensor from CO. The advantages of both polymeric coatings can be used at the same time by combining them in a multilayer approach. Another suitable coating that can be combined with PMMA is SiO₂. SiO₂ coatings have been shown to prevent oxidation of the catalytic layer in hydrogen sensors. In embodiments, PMMA is thus combined with SiO₂ to prevent both oxygen and CO diffusion. Another possibility is to combine all three coatings together, PMMA, SiO2, and PTFE. The connection between the different layers can be improved by individual activation of the already deposited coating layers by pre-sputtering with Ar ions, for example.

The PMMA coating can be deposited by different techniques on the sensor, e.g. by magnetron sputtering, or by spin-on or chemical vapor deposition techniques. Alternatively, a PMMA socket can be directly slipped on a fiber, or other sensor substrate, and sealed to it. These protective coatings according to aspects are suitable both for detection in the gas phase as well as in liquids, such as, e.g., in transformer oil.

### Detailed Description of the Figures and Embodiments

It shall be noted that the figures are not drawn to scale, and that some dimensions in the figures are exaggerated for illustrational purposes. The width of the sensor in a horizontal direction is typically much larger, for example 2 to 5 orders of magnitude, than the thickness of the sensing elements and protection layers in a vertical direction (in relation to the drawing plane).

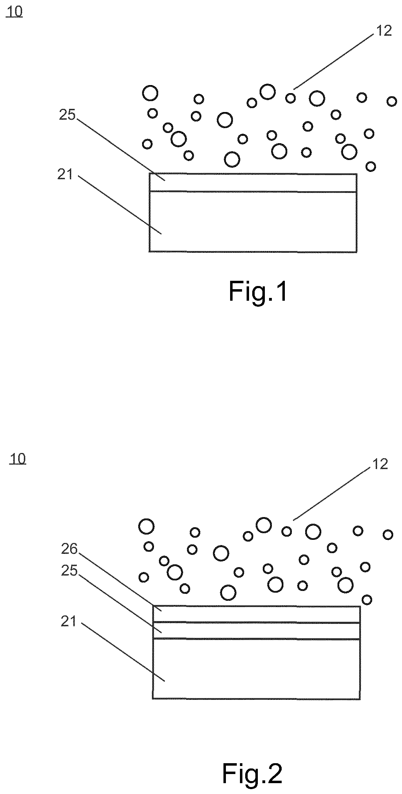

Fig. 1 shows an exemplary hydrogen sensor 10 useful for understanding the invention, for detecting hydrogen in a fluid 12 which is in physical contact with the sensor. A sensing element 21 is covered by a first protection layer 25. The sensing element typically, but not necessarily comprises a catalyst layer and a sensing layer, which can also be identical in the case of the use of Pd alloys. The hydrogen sensor 10 may typically be attached with the sensing layer to an optical fiber (not shown), through which light is directed onto the sensing element 21, while light reflected by the sensing element back into the optical fiber is guided to a light detection device where it is analysed.

The first protection layer 25 prevents direct contact of the sensing element 21 with the fluid 12, but is permeable for hydrogen. The first protection layer 25 comprises PMMA. It has a typical thickness from about 100 nm to about 3.000 nm, more typically from about 150 nm to about 2.000 nm. PMMA has been shown to have good blocking capabilities against CO in the fluid, while at the same time being highly permeable for the hydrogen which needs to pass the protection layer in order to reach the sensing layer.

Fig. 2 shows a hydrogen sensor according to the present invention wherein a first protection layer 25 of PMMA, as shown in Fig. 1, is accompanied by a second protection layer 26 which abuts the first protection layer 25 and comprises a further, different material. The second protection layer comprises one of PTFE, SiO₂, and Aluminium Oxide, typically Al₂O₃. In the case of PTFE, for example, it is known to have excellent blocking capabilities against liquids, such as water and oil. It is used in embodiments as an outermost coating layer for a hydrogen sensor for use in liquids. In the present invention, it is employed as the outermost coating layer, wherein a layer comprising PMMA is located between the sensing layer 21 and the PTFE layer.

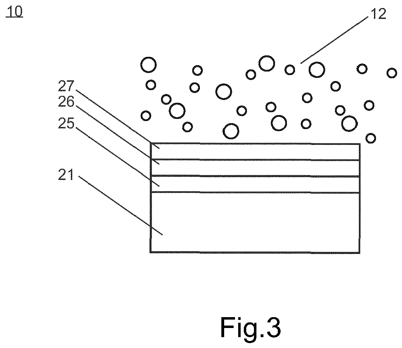

Fig. 3 shows the sensor of Fig. 2 according to embodiments, which comprises a third protection layer 27. The latter abuts the first protection layer 25 or the second protection layer 26. It may comprise one of PTFE, SiO₂, and Aluminium Oxide.

By choosing suitable material combinations for the first protection layer, and optional second protection layer and third protection layer, a hydrogen sensor with high robustness and stability for use in various environments can be designed wherein the environment may also comprise aggressive components. This is particularly interesting for environments with high temperatures, or with a significant amount of aggressive substances in the fluid in contact with the sensor. Thereby, by choosing a suitable combination, the required stability may be achieved while obtaining a high permeability for hydrogen, which is required for efficient operation of the hydrogen sensor 10.

Thereby, a protective layer of SiO₂ is suitable to prevent oxidation of the protected catalytic layer of the sensing element 21 in hydrogen sensors. Thus, when combining a first protection layer of PMMA with a second protection layer of SiO₂, it is possible to prevent both oxygen and CO diffusion from the surrounding fluid 12 to the sensing element 21. Additionally, PTFE may be employed to shield the sensor from liquid water and from oil.

The connection between the sensing element 21 and the first, second and third protection layers can be improved by activation of the already deposited layers by pre-sputtering with Ar ions. The PMMA coating can be deposited by different techniques on the sensing element. Applicable methods include magnetron sputtering, spin-on deposition or chemical vapor deposition techniques. Also, a PMMA socket can be directly slipped on the fiber and be sealed to it.

The described first, second and third protective layers, respectively coatings, are suitable both for application for detection in the gas phase as well as in liquids. Of particular interest in the present context is the application in insulation liquid of an electrical transformer, also known as transformer oil.

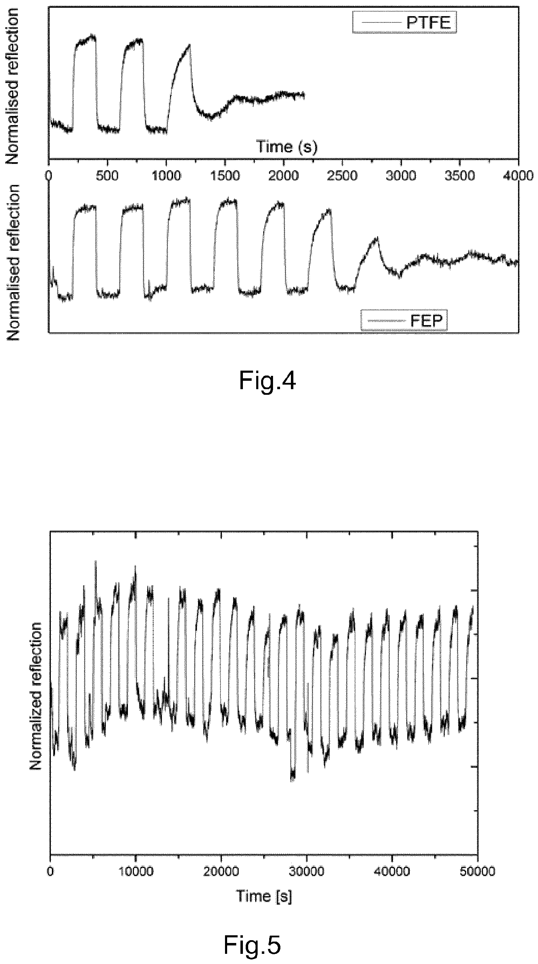

Figure 4 shows an example of the degradation of the optical response of a hydrogen sensor according to previously known technology, which is a fiber optic sensor with a sensing element wherein the sensing layer and the catalyst layer are one, and comprise a Pd alloy. The sensing layer, more precisely the catalyst layer, is coated with two different polymers for the test, namely PTFE (top in Fig. 4) and Fluorinated Ethylene Propylene (FEP), below in Fig. 4.

The optical response changes over time, when the sensor is exposed to a series of hydrogenation cycles, meaning that the hydrogen concentration above the sensor is elevated and reduced to zero alternatingly, resulting in a respective change in reflectance of the fiber optic sensor. The diagrams in Fig. 4 show the intermitting change of reflectance. Due to the presence of CO in the gas in the experiment, the sensor degradates. After several cycles, the optical response of the hydrogen sensor does not change anymore in spite of frequently changing hydrogen concentrations. Thus, in both cases, the PTFE and FEP coatings are not able to protect the sensor from poisoning from CO, even at low CO concentrations, resulting in an almost complete failure of the two conventionally coated sensors after CO exposure for about 1000 seconds in the case of PTFE coating, and after about 2500 seconds in the case of a FEP coating.

As a comparison, Figure 5 shows a result of the same test as described with respect to Fig. 4, but with the same sensor coated with PMMA according to embodiments, instead of PTFE or FEP. This sensor can be cycled with hydrogen over a long period of time without suffering a degradation of its optical sensing properties by a CO content in the applied gas mixture. This result is even more significant when regarding that the concentration of the potentially sensor poisoning CO content in the gas above the sensor was ten times higher than in the tests for the conventional sensors coated with PTFE and FEP, which were described with respect to Fig. 4. Thus, PMMA as a coating or protection layer against CO poisoning of hydrogen sensors has very good protection properties. At the same time, the diffusion of hydrogen to the sensing layer is not, or only to a negligible extent, influenced by the PMMA protection layer.

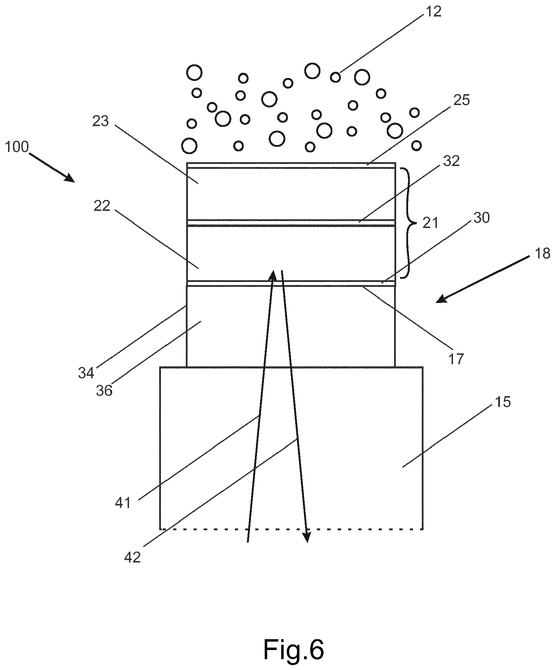

In examples not part of the present invention, the sensing element 21 may be one of a thin film, a FET, a resistive element, but according to the present invention it is a waveguide. There are a variety of sensing elements known in the art which are suitable to detect hydrogen in a fluid. These can generally be equipped with a protection layer 21 comprising PMMA, and optionally with the second protection layer 26 and third protection layer 27 in the material combinations as described before. In the following, a hydrogen sensor 100 is described, which is an example of an optical hydrogen sensor according to the present invention. In Fig. 6, an exemplary hydrogen sensor 100 according to embodiments is shown, comprising a sensing element 21 as described above with a sensing layer 22 and a catalyst layer 23. The optical sensor 100 is suitable for detecting hydrogen in a fluid 12 (schematically shown, with smaller circles representing dissolved hydrogen in the fluid, and bigger circles symbolizing CO molecules) which is in physical contact with the hydrogen sensor 100. The sensing element 21, being a multilayer, is coated to an end portion 18 of an optical fiber 15. The latter typically has an outer diameter of about 230 µm including cladding and coating, and 200 µm for the fiber core, but different diameters are also applicable. The sensing layer 22 comprises a thin film of a metal alloy, wherein the metal alloy of the sensing layer 22 may have the following, non-limiting compositions: The alloy comprises Mg, Ni, and a component M, wherein M is at least one of Zr, Ta, and Hf. The alloy has the composition MgₓNi_{y}M_{z}, wherein x is from 40 to 60, y is from 10 to 40, and z is from 10 to 40, in combinations where the numbers add up to 100. The catalyst layer 26 comprises Pd or a Pd alloy.

In embodiments, the sensing element 21 comprises a sensing layer 22 with one of the alloys with the basic composition Mg₅₂Ni₂₀Zr₂₈ and Mg₅₅Ni₂₇Ta₁₈, wherein the individual amount of the components may deviate by +-15 %. The hydrogen sensor 100 exhibits a continuous decrease of the optical reflectivity in the visible optical range, when exposed to a growing hydrogen partial pressure in a fluid 12 in contact with the hydrogen sensor 100. The hydrogen sensor 100 of Fig. 6 comprises an auxiliary layer 32 between the catalyst layer 23 and the sensing layer 22, which abuts the sensing layer 22. The auxiliary layer 32 preferably comprises Ti, which is suitable to block atoms from either neighbouring layer from diffusing into the respective other layer. A further auxiliary layer 30, also typically comprising Ti, is provided as an adhesive layer between the core 36 of the optical fiber 15 and the sensing layer 22. In embodiments, the coating layer 25 reaches over the entire multilayer that is also over the circumferential side faces of the multilayer (not shown).

The sensing element 21, which is a multilayer, is provided on an end surface 17 of the optical fiber 15, perpendicular to the longitudinal axis of the optical fiber. In embodiments, most or all layers situated consecutively on the end surface 17 overlap over the edge to cover a portion of the circumferential side face 34 of the core 36 of the optical fiber 15. In examples not part of the present invention, the sensing element 21 may also be provided exclusively on the peripheral side face 34 of the optical fiber 15. Also, the sensing element 21 may in examples not part of the present invention be provided on an optically transparent substrate (not shown) different from an optical fiber.

A first protection layer 25 comprising PMMA is protecting the sensing element 21 from CO in the fluid 12 in contact with the sensor 100. The first protection layer 25 is accompanied by a second protection layer 26 and may be accompanied by a third protection layer 27 as was described with respect to embodiments shown in Figs. 2 and 3. Hence, hydrogen sensor 100 may be protected by a multilayer protective layer comprising a combination of layers comprising PMMA, PTFE, SiO₂, and Aluminium Oxide, typically Al₂O₃.

In all embodiments, typical dimensions (i.e., a thickness parallel to the longitudinal axis of the optical fiber) for the varying layers of the multilayer are: Auxiliary layers from 2 to 7 nm, more typically from 4 to 6 nm, for example 5 nm. The sensing layer is typically from 30 to 80 nm, more typically from 40 to 70 nm, for example 60 nm thick. The catalyst layer 26 is typically from 15 nm to 50 nm, more typically from 20 to 40 nm, for example 30 nm thick. The thickness of the coating layer may vary depending on its individual setup, in particular if it comprises several layers of differing materials, as described herein. It may thus have a thickness from about 150 nm to 5 µm, more typically from 20 nm to 3 µm, for example 1 µm or 2 µm.

Schematically, an incoming light beam 41 is shown, which is reflected in the sensing layer 22 and mirrored back into the optical fiber 15 as light beam 42. The optical fiber 15 is typically a multimode fiber, the wavelength may for example be about 635 nm, but also any other wavelength in the visible optical range is applicable with the sensing layers 22 as described herein. The exact position at which the light is reflected in the sensing layer in Fig. 6 is randomly chosen for illustrational purposes only.

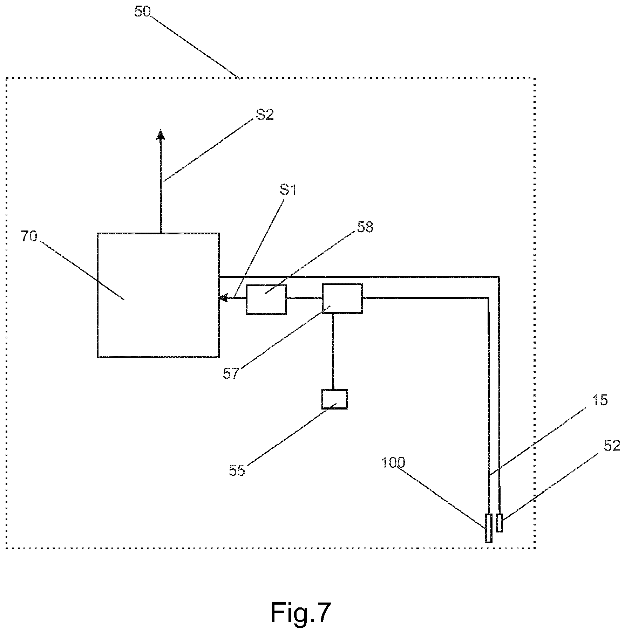

In Fig. 7, a detection system 50 for hydrogen in fluids is shown. It comprises the hydrogen sensor 100 as described above. Further, it comprises a temperature sensor 52, a light source 55, a light detection device 58, an optical splitter 57, and a control unit 70. Light from the light source 55 is coupled by the splitter 57 into the hydrogen sensor 100. In the hydrogen sensor 100, the sensing layer 22 of the sensing element 21 (both not shown) reflects a portion of the incoming light. The reflected light reflects back through the splitter 57 and is detected by light detection device 58. The latter produces an output signal S1 depending on the amplitude of the reflected light received. The control unit 70 processes the output signal S1 of the light detection device 58. It determines a hydrogen concentration and delivers a respective output signal S2. Thereby, it takes into account temperature T delivered from temperature sensor 52. Thereby, the control unit 70 makes use of data stored in a look-up table. The stored data comprises characteristic reflectance data of the optical sensor 10 for a variety of temperatures (in the range of interest, e.g. 10° C to 100 °C) and a variety of partial pressures of hydrogen in a fluid 12 (not shown) surrounding the hydrogen sensor 100. Thus, the detection system 50 provides an output signal S2 depending on the calculated hydrogen concentration in the fluid 12, which is calculated from the measured values for temperature and reflectance. Signal S2 is determined from the reflectance of the hydrogen sensor 100 and the temperature. Thereby, the output signal S2 in embodiments is typically a continuous function of the hydrogen concentration in the fluid 12. The detection system typically delivers a continuous change of the output signal S2 in dependency of a hydrogen concentration at the optical sensor, in a temperature region between 5° C and 150° C and for hydrogen partial pressures in the fluid 12 between 0,5 ppm and 5.000 ppm, more typically between 2 ppm and 3.000 ppm. Alternatively to the use of a look-up table, the hydrogen concentration may be calculated by the control unit 70 from a stored function set, taking into account at least the parameters reflectivity of the hydrogen sensor 100 and the temperature from the temperature sensor 52.

It is understood that the described optical sensors 100 and detection systems have to be characterized prior to their use in order to obtain the data mentioned above about the relation between hydrogen partial pressure, temperature and reflectance of the optical sensor.

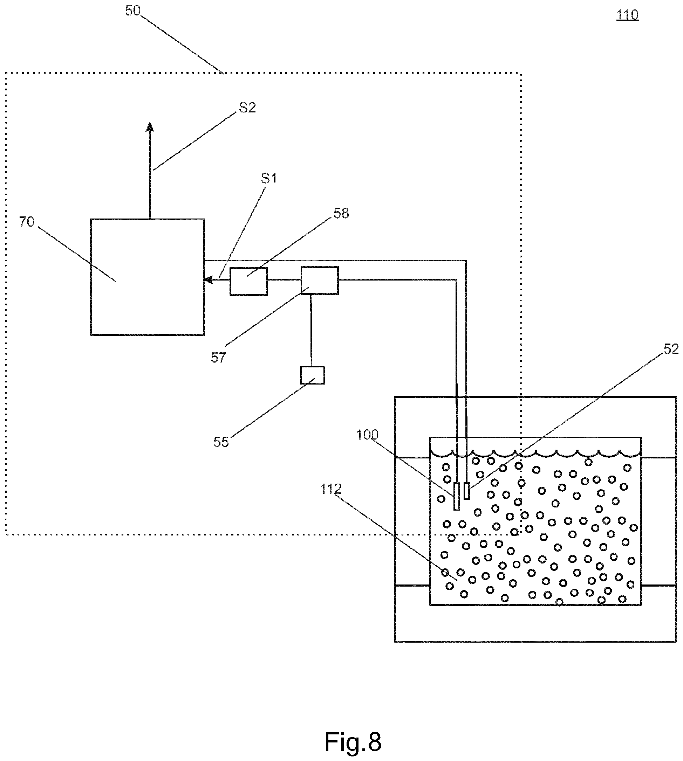

In Fig. 8, an electrical device 110 is shown. It includes a hydrogen detection system 50 as described with respect to Fig. 7. The device 110 is generally a device for electric power generation, transmission, or distribution, and more typically a power transformer or distribution transformer. It comprises an oil volume 112 for insulation and cooling purposes, in which the hydrogen sensor 100 and the temperature sensor 52 are immersed. More precisely, the end portion 18, with the sensing element 21, of the optical fiber 15 is immersed in the oil volume 112. The temperature sensor 52 is provided to measure the temperature of the oil directly at, or adjacent to the sensing element 21 of the hydrogen sensor 100. The oil is representative of the fluid 12 shown in other figures herein.

In examples not part of the present invention, the hydrogen sensor comprises at least one of a thin film, a FET, a resistive element, but according to the present invention it has a waveguide as a sensing element. Generally, nearly all types of gas sensors can be protected with one or more protection layers as described. To this end, the sensing element 21 of the sensor is placed in a sputtering device. A layer of PMMA with the intended thickness, as described above, is provided on the sensing layer. During the sputtering process, the PMMA may undergo structural modifications, which may lead to the finding that the deposited PMMA layer has properties different from standard PMMA. Also, it is understood that various other deposition technologies may be employed for providing the PMMA layer, such as spin coating or dipping the fiber end into a solution.

## Claims

1. A hydrogen sensor (10, 100) for detecting hydrogen in a fluid of oil (12) in physical contact with the sensor, comprising:
- an optical fiber (15) with a fiber core (36),
- a multilayer comprising a sensing layer (22), the multilayer being coated on an end surface (17) of the fiber core (36) perpendicular to a longitudinal axis of the optical fiber (15),
- a first protection layer (25) comprising PMMA, and
- a second protection layer (26) comprising one of SiO₂, Aluminium Oxide, and PTFE, wherein the first protection layer (25) and the second protection layer (26) are coated on the multilayer.

2. The sensor of claim 1, wherein the first protection layer (25) has a thickness from 100 nm to 3.000 nm, and is adapted to shield the sensing layer (22) from CO in the fluid (12).

3. The sensor of claims 1 or 2, comprising a third protection layer (27) abutting the first protection layer (25) or the second protection layer (26), comprising at least one of PTFE, SiO₂, and Aluminium Oxide.

4. The sensor of any preceding claim, wherein the sensing layer (22) comprises a metal alloy.

5. The sensor of any preceding claim, wherein the multilayer further comprises a catalyst layer (23), which preferably comprises Pd, provided between the sensing layer (22) and the first protection layer (25).

6. The sensor of any preceding claim, wherein the multilayer overlaps to cover a portion of the circumferential side face (34) of the fiber core (36).

7. The sensor of any preceding claim, wherein the sensing layer (22) comprises an alloy comprising Mg, Ni, and M, wherein M is at least one of Zr, Ta, and Hf.

8. The sensor of claim 7, wherein the alloy has the composition MgₓNi_{y}M_{z}, and wherein x is from 40 to 60, y is from 10 to 40, and z is from 10 to 40.

9. The sensor of any of preceding claim, wherein the sensing layer (22) comprises at least one of Mg₅₂Ni₂₀Zr₂₈, Mg₅₂Ni₂₄Zr2₄, and Mg₅₅Ni₂₇Ta₁₈.

10. The sensor of any preceding claim, further comprising at least one auxiliary layer (30, 32) abutting the multilayer, the auxiliary layer preferably comprising Ti.

11. A detection system (50) for hydrogen in fluids of oil (12), comprising a hydrogen sensor (10, 100) of any of claims 1 to 10, a temperature sensor (52), a light source (55), a light detection device (56), and a control unit (70), wherein the detection system is adapted such that light from the light source is coupled into the hydrogen sensor (10, 100), light reflected by the multilayer of the hydrogen sensor (100) is detected by the light detection device (56), and wherein the control unit (70) is adapted to process an output signal (S1) of the light detection device, to determine a hydrogen concentration, and to deliver a respective signal (S2).

12. A device (110) for electric power generation, transmission, or distribution, comprising an oil volume (112), and a detection system (50) for hydrogen in the oil according to claim 11.

13. A method for producing a hydrogen sensor (10, 100) for detecting hydrogen in a fluid of oil in physical contact with the hydrogen sensor (10, 100), the method comprising:
- providing a multilayer comprising a sensing layer (22) coated on an end surface (17) of an optical fiber core (36) perpendicular to a longitudinal axis of the optical fiber (15)
- coating, on the multilayer, a first protection layer (25) comprising PMMA, and a second protection layer (26) comprising one of SiO₂, Aluminium Oxide, and PTFE.

## Patentansprüche

1. Wasserstoffsensor (10, 100) zum Nachweisen von Wasserstoff in einem Ölfluid (12) in physischem Kontakt mit dem Sensor, umfassend:
- eine optische Faser (15) mit einem Faserkern (36),
- eine Mehrfachschicht, die eine Sensorschicht (22) umfasst, wobei die Mehrfachschicht auf eine Endoberfläche (17) des Faserkerns (36) senkrecht zu der Längsachse der optischen Faser (15) geschichtet ist,
- eine erste Schutzschicht (25), die PMMA umfasst, und
- eine zweite Schutzschicht (26), die eines von SiO₂, Aluminiumoxid und PTFE umfasst, wobei die erste Schutzschicht (25) und die zweite Schutzschicht (26) auf die Mehrfachschicht geschichtet sind.

2. Sensor gemäß Anspruch 1, wobei die erste Schutzschicht (25) eine Dicke von 100 nm bis 3.000 nm aufweist und dafür ausgelegt ist, die Sensorschicht (22) vor CO in dem Fluid (12) zu schützen.

3. Sensor gemäß Ansprüchen 1 oder 2, umfassend eine dritte Schutzschicht (27), die an die erste Schutzschicht (25) oder die zweite Schutzschicht (26) angrenzt, umfassend wenigstens eines von PTFE, SiO₂ und Aluminiumoxid.

4. Sensor gemäß einem der vorstehenden Ansprüche, wobei die Sensorschicht (22) eine Metalllegierung umfasst.

5. Sensor gemäß einem der vorstehenden Ansprüche, wobei die Mehrfachschicht ferner eine Katalysatorschicht (23) umfasst, die vorzugsweise Pd umfasst und die zwischen der Sensorschicht (22) und der ersten Schutzschicht (25) bereitgestellt ist.

6. Sensor gemäß einem der vorstehenden Ansprüche, wobei die Mehrfachschicht übergreift, um einen Teil der Umfangsseitenfläche (34) des Faserkerns (36) zu bedecken.

7. Sensor gemäß einem der vorstehenden Ansprüche, wobei die Sensorschicht (22) eine Legierung umfasst, die Mg, Ni und M umfasst, wobei M wenigstens eines von Zr, Ta und Hf ist.

8. Sensor gemäß Anspruch 7, wobei die Legierung die Zusammensetzung MgₓNi_{y}M_{z} aufweist und wobei x von 40 bis 60 beträgt, y von 10 bis 40 beträgt und z von 10 bis 40 beträgt.

9. Sensor gemäß einem der vorstehenden Ansprüche, wobei die Sensorschicht (22) wenigstens eines von Mg₅₂Ni₂₀Zr₂₈, Mg₅₂Ni₂₄Zr₂₄ und Mg₅₅Ni₂₇Ta₁₈ umfasst.

10. Sensor gemäß einem der vorstehenden Ansprüche, ferner umfassend wenigstens eine Hilfsschicht (30, 32), die an die Mehrfachschicht angrenzt, wobei die Hilfsschicht vorzugsweise Ti umfasst.

11. Nachweissystem (50) für Wasserstoff in Ölfluiden (12), umfassend einen Wasserstoffsensor (10, 100) gemäß einem der Ansprüche 1 bis 10, einen Temperatursensor (52), eine Lichtquelle (55), eine Lichtnachweisvorrichtung (56) und eine Steuereinheit (70), wobei das Nachweissystem so gestaltet ist, dass Licht aus der Lichtquelle in den Wasserstoffsensor (10, 100) eingekoppelt wird, von der Mehrfachschicht des Wasserstoffsensors (100) reflektiertes Licht von der Lichtnachweisvorrichtung (56) nachgewiesen wird, und wobei die Steuereinheit (70) dafür ausgelegt ist, ein Ausgangssignal (S1) der Lichtnachweisvorrichtung zu verarbeiten, um eine Wasserstoffkonzentration zu bestimmen und ein entsprechendes Signal (S2) auszugeben.

12. Vorrichtung (110) zur Erzeugung, Übertragung oder Verteilung von elektrischer Energie, die ein Ölvolumen (112) und ein Nachweissystem (50) für Wasserstoff in dem Öl gemäß Anspruch 11 umfasst.

13. Verfahren zur Herstellung eines Wasserstoffsensors (10, 100) zum Nachweisen von Wasserstoff in einem Ölfluid in physischem Kontakt mit dem Wasserstoffsensor (10, 100), wobei das Verfahren umfasst:
- Bereitstellen einer Mehrfachschicht, die eine Sensorschicht (22) umfasst, die auf eine Endoberfläche (17) eines optischen Faserkerns (36) senkrecht zu der Längsachse der optischen Faser (15) geschichtet ist,
- Aufschichten einer ersten Schutzschicht (25), die PMMA umfasst, und einer zweiten Schutzschicht (26), die eines von SiO₂, Aluminiumoxid und PTFE umfasst, auf die Mehrfachschicht.

## Revendications

1. Un capteur d'hydrogène (10, 100) pour la détection d'hydrogène dans un fluide constitué d'huile (12) en contact physique avec le capteur, comprenant :
- une fibre optique (15) comprenant un coeur de fibre (36),
- une multicouche comprenant une couche de détection (22), la multicouche étant appliquée en revêtement sur une surface d'extrémité (17) du coeur de fibre (36) perpendiculairement à un axe longitudinal de la fibre optique (15),
- une première couche de protection (25) comprenant du PMMA et
- une deuxième couche de protection (26) comprenant l'un du SiO₂, de l'oxyde d'aluminium et du PTFE, la première couche de protection (25) et la deuxième couche de protection (26) étant appliquées en revêtement sur la multicouche.

2. Le capteur selon la revendication 1, dans lequel la première couche de protection (25) a une épaisseur de 100 nm à 3 000 nm et est conçue pour protéger la couche de détection (22) du CO présent dans le fluide (12).

3. Le capteur selon les revendications 1 ou 2, comprenant une troisième couche de protection (27) contiguë à la première couche de protection (25) ou la deuxième couche de protection (26), comprenant au moins l'un du PTFE, du SiO₂ et de l'oxyde d'aluminium.

4. Le capteur selon une quelconque revendication précédente, dans lequel la couche de détection (22) comprend un alliage métallique.

5. Le capteur selon une quelconque revendication précédente, dans lequel la multicouche comprend en outre une couche de catalyseur (23), qui de préférence comprend du Pd, disposée entre la couche de détection (22) et la première couche de protection (25).

6. Le capteur selon une quelconque revendication précédente, dans lequel la multicouche chevauche une partie de la face latérale circonférentielle (34) du coeur de fibre (36) pour la recouvrir.

7. Le capteur selon une quelconque revendication précédente, dans lequel la couche de détection (22) comprend un alliage comprenant Mg, Ni et M, M étant au moins l'un de Zr, Ta et Hf.

8. Le capteur selon la revendication 7, dans lequel l'alliage a la composition MgₓNi_{y}M_{z} et dans lequel x va de 40 à 60, y va de 10 à 40 et z va de 10 à 40.

9. Le capteur selon une quelconque revendication précédente, dans lequel la couche de détection (22) comprend au moins l'un de Mg₅₂Ni₂₀Zr₂₈, Mg₅₂Ni₂₄Zr₂₄ et Mg₅₅Ni₂₇Ta₁₈.

10. Le capteur selon une quelconque revendication précédente, comprenant en outre au moins une couche auxiliaire (30, 32) contiguë à la multicouche, la couche auxiliaire de préférence comprenant du Ti.

11. Un système de détection (50) pour de l'hydrogène dans des fluides constitués d'huile (12), comprenant un capteur d'hydrogène (10, 100) selon l'une quelconque des revendications 1 à 10, une sonde de température (52), une source de lumière (55), un dispositif de détection de lumière (56) et une unité de commande (70), le système de détection étant conçu de façon telle que la lumière provenant de la source de lumière est couplée dans le capteur d'hydrogène (10, 100), la lumière réfléchie par la multicouche du capteur d'hydrogène (100) est détectée par le dispositif de détection de lumière (56) et l'unité de commande (70) étant conçue pour traiter un signal de sortie (S1) du dispositif de détection de lumière, pour déterminer une concentration d'hydrogène et pour fournir en sortie un signal correspondant (S2).

12. Un dispositif (110) pour la production, la transmission ou la distribution d'énergie électrique, comprenant un volume d'huile (112) et un système de détection (50) pour l'hydrogène dans l'huile selon la revendication 11.

13. Un procédé pour la production d'un capteur d'hydrogène (10, 100) pour la détection d'hydrogène dans un fluide constitué d'huile en contact physique avec le capteur d'hydrogène (10, 100), le procédé comprenant :
- la disposition d'une multicouche comprenant une couche de détection (22) en revêtement sur une surface d'extrémité (17) d'un coeur (36) de fibre optique perpendiculairement à un axe longitudinal de la fibre optique (15),
- l'application en revêtement, sur la multicouche, d'une première couche de protection (25) comprenant du PMMA et d'une deuxième couche de protection (26) comprenant l'un du SiO₂, de l'oxyde d'aluminium et du PTFE.
